# EUROPEAN PATENT SPECIFICATION

(11) **EP 2 995 689 B1**
(45) Date of publication and mention of the grant of the patent: **13.09.2017**
(21) Application number: 14461567.1
(22) Date of filing: 11.09.2014
(51) Int. Cl.: C12Q 1/68

(54) **Stratification of B-cell lymphoma cases using a gene expression signature**
Schichtung von B-Zell-Lymphomfällen mittels Genexpressionssignatur
Stratification de cas de lymphome à cellules B à l'aide d'une signature d'expression génique

(43) Date of publication of application: 16.03.2016
(73) Proprietor: Warszawski Uniwersytet Medyczny, 02-091 Warszawa (PL)
(72) Inventor: Gaj, Pawel, 02-776 Warszawa (PL); Zagozdzon, Radoslaw, 05-080 Truskaw (PL)
(74) Representative: Patpol Kancelaria Patentowa Sp. z o.o.

(56) References cited:
- US-A1- 2005 164 231
- HUMMEL M ET AL: "A biologic definition of Burkitt's lymphoma from transcriptional and genomic profiling", NEW ENGLAND JOURNAL OF MEDICINE, MASSACHUSETTS MEDICAL SOCIETY, US, vol. 354, no. 23, 8 June 2006 (2006-06-08) , pages 2419-2430, XP002619995, ISSN: 0028-4793, DOI: 10.1056/NEJMOA055351
- SANDEEP S DAVE ET AL: "T Molecular Diagnosis of Burkitt's Lymphoma A BS TR AC T", N ENGL J MED, vol. 354, 8 June 2006 (2006-06-08), pages 2431-42, XP055171087,
- ELLEN LEICH ET AL: "Diagnostic and prognostic significance of gene expression profiling in lymphomas", APMIS, vol. 115, no. 10, 1 October 2007 (2007-10-01), pages 1135-1146, XP055171089, ISSN: 0903-4641, DOI: 10.1111/j.1600-0463.2007.apm_867.xml.x

## Description

The present invention relates to stratification of B-cell lymphoma cases using a newly identified gene expression signature of 11 genes. In particular, the invention relates to the use of gene expression signature consisting of SLC25A24, BIK, C9orf64, PRKAR2B, LGALS1, LHFP, IGHM, TAF9, PLCL2, TFDP2 and LIG4 for differentiation between Burkitt's lymphoma (BL) and diffuse large B-cell lymphoma (DLBCL). Moreover, the invention provides qPCR kit for differentiation between BL and DLBCL and a method of in vitro differentiation between BL and DLBCL.

### Prior Art

Non-Hodgkin lymphoma (NHL) is a heterogeneous group of tumors displaying a diversity of biological phenotypes, clinical behaviors and treatment sensitivities. Although complete response rates of over 50% can be observed in patients receiving chemotherapy alone, a significant number of patients relapse, and develop resistance to further treatment. The type of NHL strongly predicts therapeutic outcomes and therefore, correct diagnosis is of paramount importance. Currently, immunohistochemistry (IHC) along with cytomorphology and cytogenetics are routinely used diagnostic procedures in NHL. These procedures are time-, cost- and labor-consuming, and require highly trained personnel for the analysis and interpretation of the results. Moreover, the IHC staining procedures are prone to variations between batches of reagents, different tissue preservation techniques, duration of tissue fixation, or even result from minimal changes in the duration of incubation with chromogens. Also, evaluation of staining intensity is a critical factor that may hinder reproducibility of results in tissue-based phenotypic lymphoma studies. These problems might be especially pertinent to the appropriate diagnostic classification of two lymphoma types: Burkitt's lymphoma (BL) and diffuse large B-cell lymphoma (DLBCLs). The clinical course of these two malignancies significantly differs, and despite multiple studies, the biological grounds for this remain unclear.

Burkitt's lymphoma (BL) defines a distinct group of B-cell malignancies that are primarily found in the germinal centers (secondary follicles) of the peripheral lymph nodes. The World Health Organization (WHO) defines the BL as an aggressive non-Hodgkin lymphoma (NHL) originating from the mature stage B lymphocytes. This lymphoma type is characterized by high rates of cell proliferation as measured by the Ki-67 protein expression, and show cytogenetic changes, usually translocations of the chromosomal region containing the MYC gene with the locus of the IGH@ (immunoglobulin heavy chain locus), t(8;14)(q24;q32), but also t(2;8), t(8;22), t(14;18), t(3q27). Importantly, the changes affecting the MYC are not the only genetic alterations identified so far in BL, and identification of the pathogenic factors that would be exclusively found in the BL, but not in other types of NHL has not been successful. This also holds true for the diagnostic criteria focusing the immunophenotyping of the BL.

The potential for diagnostic differentiation between the BL and the DLBCLs is of clinical relevance.

Although the criteria for the well-defined BL and DLBCLs have been described, there are numerous cases of lymphoma, which show morphological features characteristic for both BL and DLBCLs, and therefore cannot be easily classified to either of the two lymphoma subtypes. These cases are frequently described as atypical Burkitt's lymphomas (aBL) or Burkitt-like lymphomas (BLL) [Thomas DA et al., Burkitt lymphoma and atypical Burkitt or Burkitt-like lymphoma: should these be treated as different diseases? Curr Hematol Malig Rep. March 2011;6(1):58-66]. In the 2008 World Health Organization (WHO) classification system, these borderline cases are referred to as "B-cell lymphoma, unclassifiable, with features intermediate between diffuse large B-cell lymphoma and Burkitt lymphoma.", as described in [Leoncini L, Raphael M, Stein H, Harris NL, Jaffe ES, Kluin PM. Burkitt Lymphoma. In: Swerdlow SH, Campo E, Harris NL, et al., eds. WHO Classification of Tumours of Haematopoietic and Lymphoid Tissues. 4th ed. Lyon, France: IARC Press; 2008:262-4.], as well as in [Kluin PM, Harris NL, Stein H, Leoncini L. B-cell lymphoma, unclassifiable, with features intermediate between diffuse large B-cell lymphoma and Burkitt lymphoma. In: Swerdlow SH, Campo E, Harris NL, et al., eds. WHO Classification of Tumours of Haematopoietic and Lymphoid Tissues. 4th ed. Lyon, France: IARC Press; 2008:265-7.].

According to the widely accepted clinical practice, precise diagnostics of BL should be based on laborious evaluation of a wide selection of specific antibodies, as well as a detailed cytogenetic and molecular screening, which besides the Ki-67 expression also evaluate the expression of the CD10, BCL6, BCL2 [Bellan C et al., Burkitt lymphoma versus diffuse large B-cell lymphoma: a practical approach. Hematol Oncol. June 2010;28(2):53-6]. The difficulties in correct classification of those atypical cases of BL frequently translate into misguided therapeutic decisions and therefore can be reflected by significantly reduced patient survival rates. All these diagnostic uncertainties explain why WHO does not recognize aBL as a distinct disease entity. Hence, the aBL cases remain in a so-called "grey zone" [Salaverria I et al., The gray zone between Burkitt's lymphoma and diffuse large B-cell lymphoma from a genetics perspective. J Clin Oncol Off J Am Soc Clin Oncol. 10 May 2011;29(14):1835-43] between classic BL and DLBCL, defining not only a diagnostic issue, but also a scientific challenge. The importance of the described classification is underscored even further due to the fact that the two types of lymphoma require quite different therapeutic strategies: aggressive therapeutic plans for the patients diagnosed with either aBL or classic BL, and somewhat milder chemo- or chemoimmunotherapy involving typically used cyclophosphamide, doxorubicin, vincristine, and prednisone (CHOP) in the case of DLBCL patients [Dave SS et al., Molecular diagnosis of Burkitt's lymphoma. N Engl J Med. 8 June 2006;354(23):2431-42].

Comparative transcriptomic approaches supported by complex bioinformatics have defined one of the emerging strategies addressing specific classification of BL and DLBCL. These studies focus mainly on the gene expression data obtained from the biopsy tissue material derived from patient cohorts including BL, aBL and DLBCL cases [Dave SS et al., supra, Hummel M et al., A biologic definition of Burkitt's lymphoma from transcriptional and genomic profiling. N Engl J Med. 8 June 2006;354(23):2419-30]. The main purpose of such studies is the identification of a gene signature(s) that would allow for a specific and sensitive classification of the studied lymphoma cases. The gene signatures also give the chance to discover genes, which may play an important role in the pathogenesis or progression of the studied malignancies or their subtypes.

Importantly, the analyses conducted so far by other research teams were based on the biopsy tissue material. These approaches have both advantages and shortcomings. The obvious downside of studying the tissue samples in the transcriptomic context is (i) the fact that the clinical diagnosis for the analyzed cases was made in each of the patients ad hoc, which could introduce a bias related to the diagnostic criteria discrepancies and (ii) the biopsy tissue sample specimens contain variable proportions of the cancer cells, a factor which has a strong potential to decrease power of the study. Both of these factors are likely to effectively bias the resulting gene signature composition, and can lead to a lesser degree of confidence in the discovery part of a study. Thus, a large number of transcripts within the gene signature is necessary to properly stratify the cases. These factors hamper the potential use of such signatures in clinical practice. Methods for identifying, diagnosing and predicting survival in a lymphoma or lymphoproliferative disorder, including BL and DLBCL, on the basis of gene expression patterns were disclosed in WO 2008/013910. This publication also discloses a method for differentiation between BL and DLBCL using expression levels of c-myc and c-myc target genes.

Predictive biomarkers for NHL, including DLCBCL, have been disclosed in publication US 2012/0134986. The method disclosed therein utilizes a measurement of a single gene expressed by tumor cells (LMO2) and a single gene expressed by the immune microenvironment (TNFRSF9) to determine overall survival in patients with NHL.

In EP 2 416 270 an analytical strategy using selection of biologically relevant genes is presented. The said strategy aims to make microarray data easier to interpret by obtaining individual gene expression levels from cells obtained from one or more patients with a disease or condition; recording the expression value for each gene in a table that is divided into clusters; iteratively selecting gene expression values for one or more transcriptional modules; removing the selected genes from the analysis; and repeating the process of gene expression value selection for genes that cluster in a sub-fraction of the diseases or conditions; and iteratively repeating the generation of modules. Also publication US 2005/164231 and Ellen Leich et al. (Diagnostic and prognostic significance of gene expression profiling in lymphomas.APMIS, vol. 115, no. 10, 1 October 2007, pages 1135-1146) describe gene expression profiles that are used to analyse lymphomas. However, none of the above identified publications identifies a particular gene signature useful for differentiation of between BL and DLBCL.

### Summary of invention

In order to precisely define the molecular differences between BL and DLBCL, the Inventors of the present invention carried out a robust bioinformatics analysis of the transcriptomic data using an extensive panel of cell lines originating from these tumors, as well as a clinical set of biopsies. As the result, a set of 11 genes (i.e. SLC25A24, BIK, C9orf64, PRKAR2B, LGALS1, LHFP, IGHM, TAF9, PLCL2, TFDP2, and LIG4 genes) has been identified, which were strongly differentially expressed between BLs and DLBCLs, and the use of which in clinical practice allows for rapid, semi-automated and cost-effective stratification of B-cell lymphomas into two groups and 'flagging' the patients requiring the most intensified care.

The present invention is based on the bioinformatics approach that allows to overcome the aforementioned shortcomings. The Inventors used a training set consisting of 29 cell lines classified as B-cell lymphoma originating from the mature B lymphocytes that included a panel of 11 BL (BL41, BL70, CA46, DAUDI, EB1, EB2, GA10, NAMALWA, P3HR1, RAJI, ST486) and 18 DLBCL (A3KAW, A4FUK, DB, DOHH2, KARPAS422, NUDHL1, OCILY10, OCILY19, OCILY3, PFEIFFER, SUDHL10, SUDHL4, SUDHL5, SUDHL6, SUDHL8, TOLEDO, U937, WSUDLCL2) cell lines. The significant advantage of this approach comes from the fact that, as opposed to the clinical biopsy samples, cell lines consist of pure populations of cancer cells and therefore the differential expression effects are unlikely to be masked by the variable amounts of healthy tissue cells, usually sampled together with the cancer cells in the biopsy material. Secondly, the cell lines are by their nature very well described in terms of their phenotypes, and their sub-classification has been validated multiple times by independent research groups leaving virtually no chance for their miss-classification. Hence in this way approach used by the Inventors substantially gains in terms of the discovery power as compared with the previously described studies.

To obtain the data set, the gene expression was measured and raw Affymetrix CEL files were converted to a single value for each probe set using Robust Multi-array Average (RMA) and normalized using quantile normalization. Either the original Affymetrix GeneChip^{®} Human Genome U133 Plus 2.0 CDF file or a redefined custom CDF file (ENTREZG - v15) was used for the summarization [Dai M et al., Evolving gene/transcript definitions significantly alter the interpretation of GeneChip data. Nucleic Acids Res. 1 January 2005;33(20):e175-e175].

The Inventors have also validated the results obtained at the bioinformatics stage using RT-qPCR method. Moreover, they have carried out a representative analysis showing that the gene expression signature selected by them can be used to calculate the probability of BL diagnosis.

The invention provides therefore the use of gene expression signature consisting of SLC25A24, BIK, C9orf64, PRKAR2B, LGALS1, LHFP, IGHM, TAF9, PLCL2, TFDP2 and LIG4 for differentiation between Burkitt's lymphoma (BL) and diffuse large B-cell lymphoma (DLBCL).

The term "gene expression signature" refers to a pattern of coordinately expressed genes, which are expressed specifically and reflect biological aspects of either BL or DLBCL.

The term "differentiation between Burkitt's lymphoma (BL) and diffuse large B-cell lymphoma (DLBCL)" and/or "differentiation between BL and DLBCL" refers to classification of the sample as derived from either BL or DLBCL patient, accordingly.

The set of genes identified herein as gene signature for differentiation between BL and DLBCL can be used for preparation of the suitable qPCR kit. The set qPCR kit for differentiation between BL and DLBCL of the invention comprises primers for the combination of the genes consisting of SLC25A24, BIK, C9orf64, PRKAR2B, LGALS1, LHFP, IGHM, TAF9, PLCL2, TFDP2 and LIG4 and primers for at least one reference gene. Preferably, the said kit comprises primers selected from a group consisting of SEQ NO ID: 1 to 8.

The gene expression signature disclosed herein, can be also used to design a microarray comprising probes for identification of SLC25A24, BIK, C9orf64, PRKAR2B, LGALS1, LHFP, IGHM, TAF9, PLCL2, TFDP2 and LIG4 genes and probes for at least one reference gene. The methods for designing and producing microarrays for suitable genes are well known in the art (see for example WO 2008/013910). There is a wide range of commercially available microarrays (for example, from Agilent Technologies, Inc. and Affymetrix, Inc.) that can be customized to enable for precise determination of gene expression levels for selected set of genes according to the invention.

The invention also provides in vitro method of differentiation between Burkitt's lymphoma (BL) and diffuse large B-cell lymphoma (DLBCL), characterized in that it comprises the steps of:
a) obtaining the gene expression profile consisting of the following genes: SLC25A24, BIK, C9orf64, PRKAR2B, LGALS 1, LHFP, IGHM, TAF9, PLCL2, TFDP2, and LIG4, for samples from a training group of patients diagnosed as suffering from BL or DLBCL, wherein the gene expression profile is normalized based on the expression level of at least one reference gene;
b) obtaining the gene expression profile consisting of the following genes: SLC25A24, BIK, C9orf64, PRKAR2B, LGALS 1, LHFP, IGHM, TAF9, PLCL2, TFDP2, and LIG4, for a sample from a patient suffering from non-Hodgkin lymphoma (NHL) to be differentiated as either BL and DLBCL, wherein the gene expression profile is normalized based on the expression level of at least one reference gene and wherein the processes of obtaining the gene expression profile and its normalization are carried out in the same way as in step (a);
c) calculating the probability of BL and DLBCL diagnosis for a sample from step (b) based on an algorithm designed in reference to the data obtained in step (a).

The reference gene is any gene that allows for determination fold-differences in expression of the genes in the gene expression signature of the invention. Human ACTB - actin, beta gene - is an example of a suitable reference gene that can be used according to the invention.

Algorithms suitable for calculating the probability of diagnosis other than the algorithm proposed by inventors in the Example 4, as well as methods for designating such algorithms are well known in the art, see for example WO 2008/013910 or Hummel M et al. [supra].

### Description of the drawings

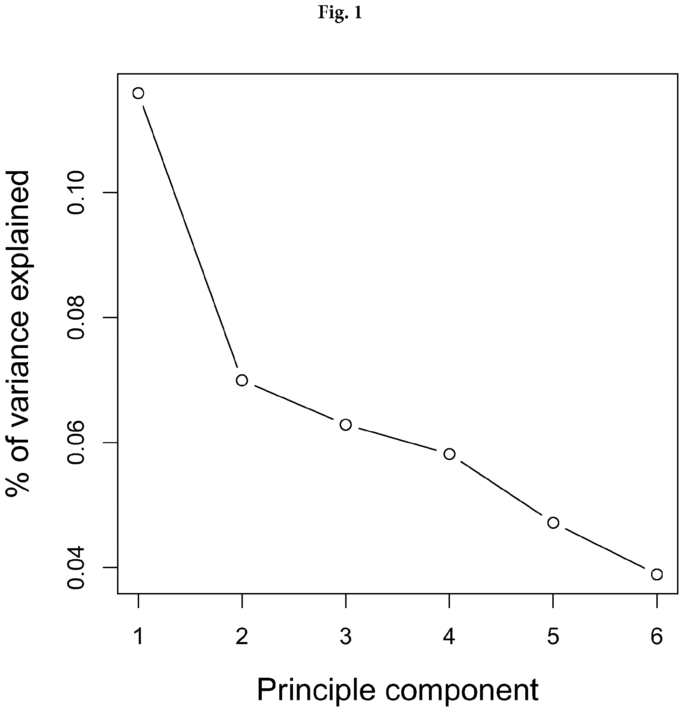

Figure 1 presents a plot of percentage of the global variance for the first six principal components in the studied lymphoma cell lines. The principle component analysis (PCA) was based on the log2 values of the expression signal for the genes assayed on the Affymetrix GeneChip^{®} Human Genome U133 Plus 2.0 arrays. The first principle component PC1 could be attributed a significantly higher share of the variance describing the data as compared to any other principle components respectively.

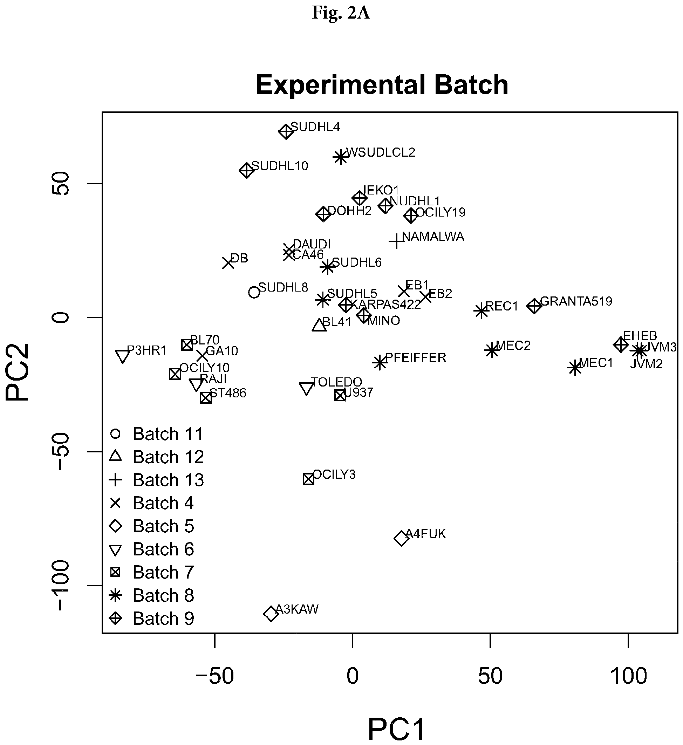

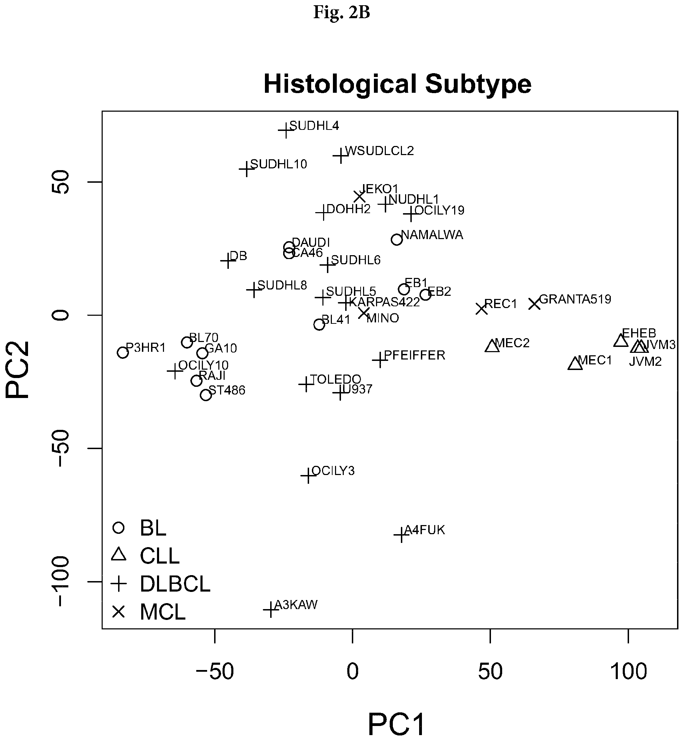

Figure 2 presents principle component analysis plots for the first and second principle components. A: patterns of clustering did not show any clear correlation with the experimental batches the samples data was obtained from; B: the clustering in coordinates of the first two principle components showed correlation with the histological subtypes of the studied lymphoma types. This effect was especially clear for the contribution of the histological subtypes to the variance explained by the first principle component (PC1).

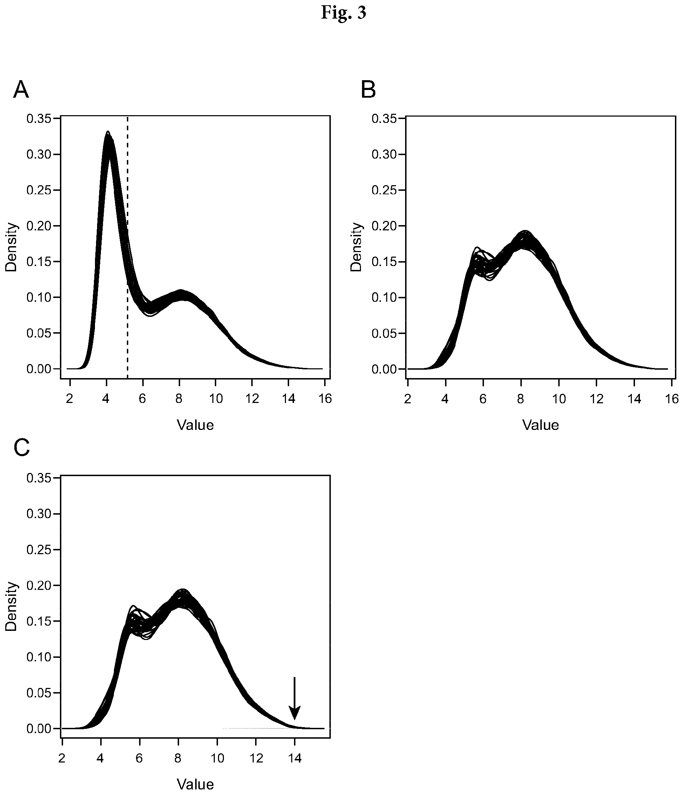

Figure 3 presents the effects of filtration of the set of genes assayed using the Affymetrix GeneChip^{®} Human Genome U133 Plus 2.0 arrays. A: the initial profile of the normalized expression signal (log2) with the threshold level marked for the background signal level based on the 95 percentile of the expression signal measured for the genes localized on the chromosome Y in the female samples. B: the expression signal distribution across all of the samples after the background signal filtration; C: the effect of the second step of filtration removing genes which showed very low variance (SD/mean < 2) of the expression signal distribution across the studied samples. The low variance filtration effect could be observed towards the far-right end of the expression signal density plot (marked with an arrow) which indicated a possibility that a subset of strongly expressed (transcribed) genes in the tissue cells could not be precisely measured on the array due to the possible saturation of the respective probes present for those genes on the micro-array surface. Those genes were removed from the data set before further statistical processing.

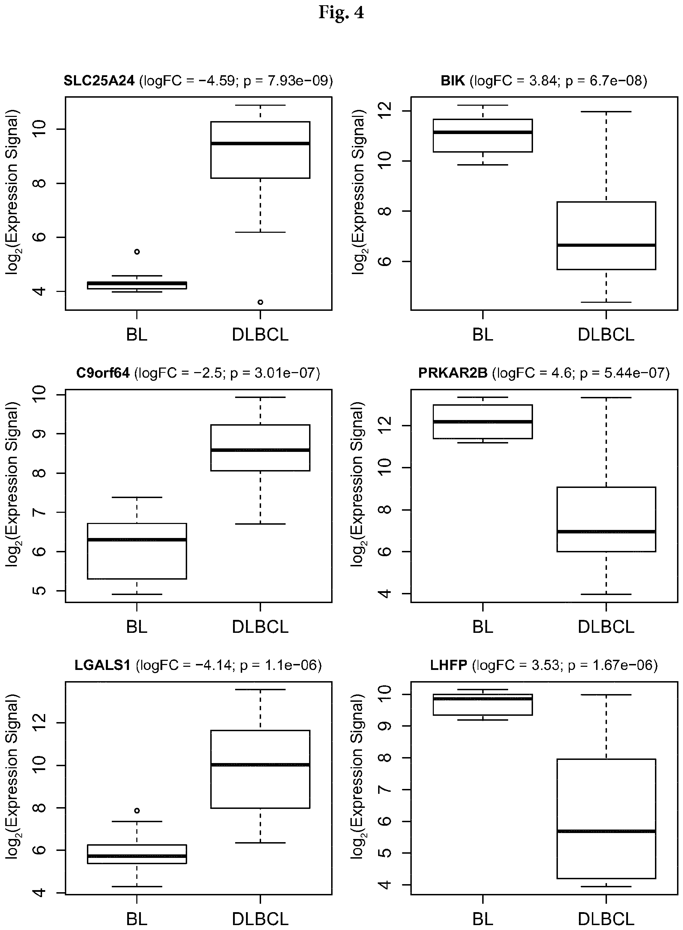

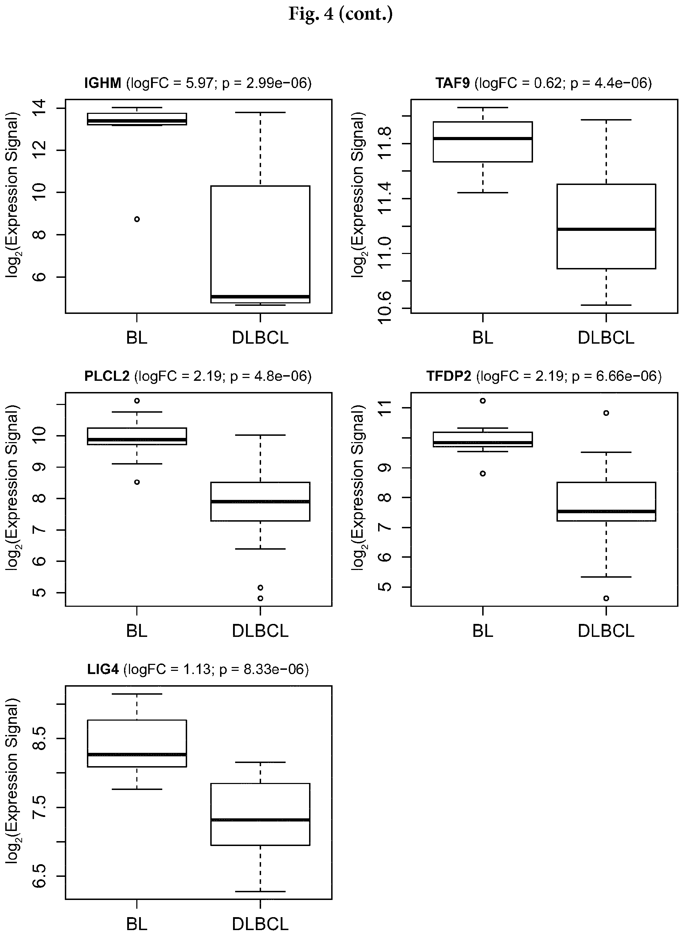

Figure 4 presents normalized gene expression levels for the 11 genes most significantly differentially expressed between the studied histological types of B-cell lymphoma cell lines. The identified signature allows for precise discrimination between BLs and the DLBCLs.

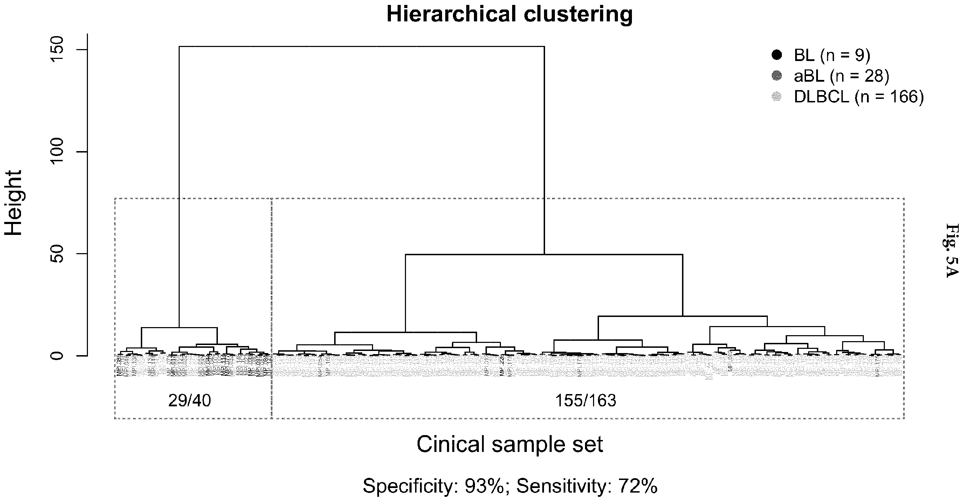

Figure 5A presents hierarchical clustering carried out using the gene expression data set obtained for the clinical lymphoma patient cohort, considering a gene signature consisting of genes common for the newly identified signature and the signature published by Hummel et al.;

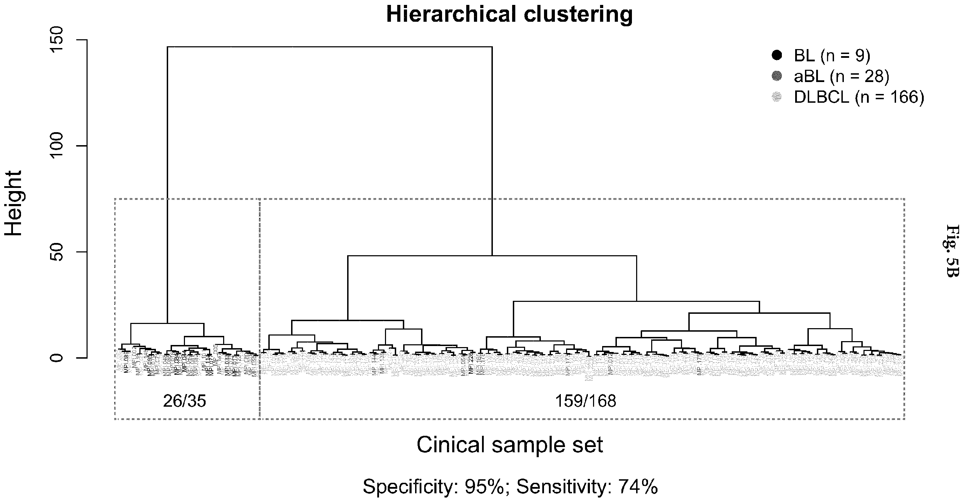

Figure 5B presents hierarchical clustering based on the full 11 gene signature of the present invention selected in the present cell line panel-based screening.

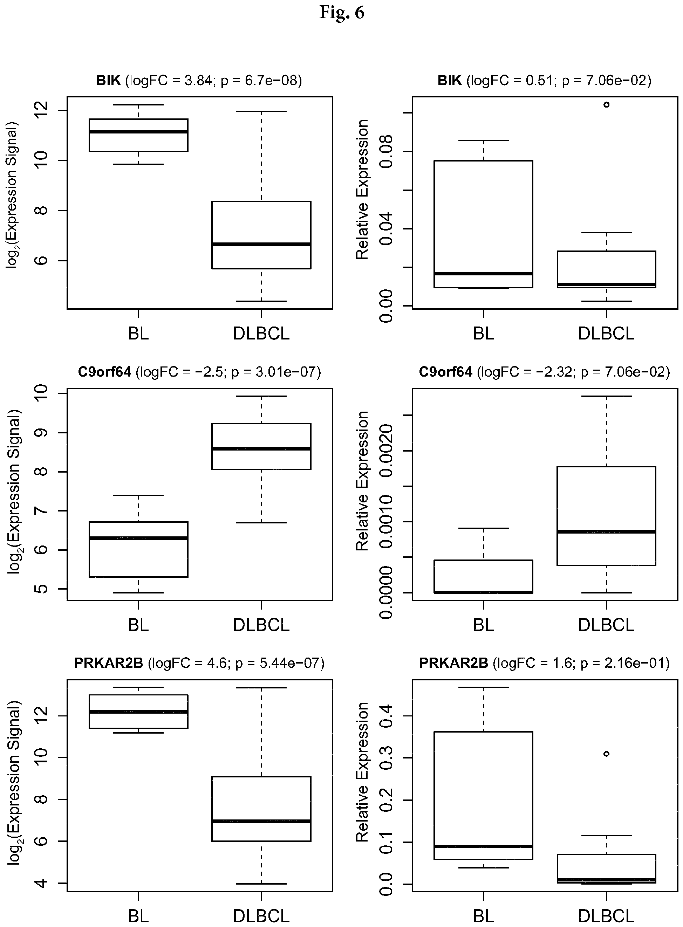

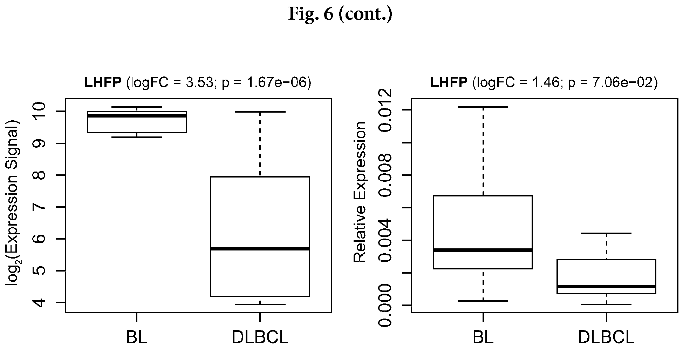

Figure 6 presents validation of the differential expression for the selected genes from the identified signature. Left plots show the results of the initial bioinformatics analysis, while the right plots indicate results for the corresponding genes determined for the independent set of B-cell lymphoma cell lines by the RT-qPCR technique.

### Example 1 - Gene selection

### Quality control (QC) of the training data set

Before the data filtering the gene expression data set was subjected to a quality control step, which included principle component analysis of the data to identify outlier samples and rule out possible batch effects related to the laboratory experiments, which the analyzed data was obtained from. The initial step of this analysis identified the percentage of the total variance, which could be attributed to the respective principle components. The principle component analysis (PCA) was based on the log2 values of the expression signal for the genes assayed on the Affymetrix GeneChip^{®} Human Genome U133 Plus 2.0 arrays. The first principle component PC1 could be attributed a significantly higher share of the variance describing the data as compared to any other principle components respectively. As shown on Figure 1 the first principle component explained substantially higher percentage of the global variance represented in the analyzed data compared to the percentages represented by all the other principle components respectively. In order to determine the possible factor which could be predominantly responsible for the observed variance we plotted the coordinates of the first and second principle components computed for each of the samples and applied symbol schemes to reflect available annotations for the analyzed samples. As indicated on Figure 2A, patterns observed on the PCA plots did not show evidence of unwanted correlation with the experimental batches the data for the respective samples were obtained from (i.e. patterns of clustering did not show any clear correlation with the experimental batches). Conversely, the correlation of the symbol scheme with the histological subtypes of the analyzed cell lines were much more evident. The most evident clustering of the samples was clear for the respective histological subtypes in the scale of the first principle component. This effect was very desirable as it could be used to prove that the most significant variation present in the analyzed data set was mirroring the relevant biological effects of the histological subtypes of the cell lines rather than the confounding experimental method errors possibly introduced by technical aspects of the laboratory procedures. No evidence of outlier samples was observed in the studied data set particularly within the scale of the first principle component PC1 (Figure 2A and B).

### Filtration of the training data set

To strengthen power of statistical testing of differences in expression between the studied cell line types the genes which demonstrated very low expression signal in the microarray experiment were removed from the further statistical processing. In order to perform this filtering, we identified the 95^{th} percentile of the expression signal of the genes annotated to the chromosome Y in the female samples (Figure 3A). This has given an estimation of the signal level characteristic for the background signal of the analyzed series of microarray experiments (Figure 3B). Finally the cell line gene expression data set was filtered to maintain only those genes that showed gene expression signal levels greater than the estimated background signal in at least 40% of samples (Figure 3C). Although the number of genes which were removed from the data set was quite substantial (8281 genes removed from the initially measured 18926 genes) those genes were uninformative for the purpose of the subject analysis due to being very unlikely differentially expressed between studied cell line types.

The second step of filtration of the dataset removed genes which showed very low variance of expression signal distribution between all studied samples (SD/mean < 2). Interestingly, as shown in Figure 3C, the effect of this filtration was very weak and could be observed primarily for the genes which demonstrated extremely high expression signals - possibly reaching the saturation levels of the dynamic range of the microarray used. The small number of genes removed in the second step of filtration (51 genes) were also very unlikely to show differential expression between the studied samples. The number of genes subjected to further statistical processing included the final number of 10594 genes.

### Selection of the differentially expressed genes

The Inventors have identified a gene expression signature in the context of lymphomas originating from the mature stage B lymphocytes (Figure 4 and Table 1). Interestingly, some of the genes were exclusively regulated in the BL cell lines and not in the CLL and/or MCL lymphomas (data not presented).

Using the two-tailed t-test we singled-out the gene signature to select a small number of 11 genes with strongly differential expression levels (FDR < 0.01) allowing for precise discrimination between the BL and DLBCL cell lines. There were four genes with a previously identified role in BL pathogenesis, in addition to seven newly identified ones, among the allocated genes. The summary information on the Affymetrix probesets collapsing into specific gene categories has been included in the Table 2.

**Table 1 Genes selected as differentially expressed between BL and DLBCL cell lines at FDR < 0.01.**

| **Entrez ID** | **Symbol** | **Name** | **Log2FC** | **P-value** | **FDR** |
|---|---|---|---|---|---|
| 29957 | SLC25A24 | solute carrier family 25 (mitochondrial carrier; phosphate carrier), member 24 | -4.59 | 7.93E-09 | 8.57E-05 |
| 638 | BIK | BCL2-interacting killer (apoptosis-inducing) | 3.84 | 6.70E-08 | 3.62E-04 |
| 84267 | C9orf64 | chromosome 9 open reading frame 64 | -2.50 | 3.01E-07 | 1.08E-03 |
| 5577 | PRKAR2B | protein kinase, cAMP-dependent, regulatory, type II, beta | 4.60 | 5.44E-07 | 1.47E-03 |
| 3956 | LGALS1 | lectin, galactoside-binding, soluble, 1 | -4.14 | 1.10E-06 | 2.38E-03 |
| 10186 | LHFP | lipoma HMGIC fusion partner | 3.53 | 1.67E-06 | 3.01E-03 |
| 3507 | IGHM | immunoglobulin heavy constant mu | 5.97 | 2.99E-06 | 4.61E-03 |
| 6880 | TAF9 | TAF9 RNA polymerase II, TATA box binding protein (TBP)-associated factor, 32kDa | 0.62 | 4.40E-06 | 5.77E-03 |
| 23228 | PLCL2 | phospholipase C-like 2 | 2.19 | 4.80E-06 | 5.77E-03 |
| 7029 | TFDP2 | transcription factor Dp-2 (E2F dimerization partner 2) | 2.19 | 6.66E-06 | 7.20E-03 |
| 3981 | LIG4 | ligase IV, DNA, ATP-dependent | 1.13 | 8.33E-06 | 8.19E-03 |

| | | | | | |
|---|---|---|---|---|---|
| *FC: fold change; BL: Burkitt's lymphoma; DLBCL: Diffuse Large B*-cell Lymphoma | | | | | |

**Table 2 Mapping of the summarized ENTREZ gene probesets onto the Affymetrix HGU133Plus2 chip for the selected genes differentially expressed between the Burkitt and Diffuse Large B-cell Lymphomas. Chromosomal positions have been listed according to the reference genome GRCh37/hg19.**

| **Gene Name** | **Probe Set Name** | **Chr** | **Chr Strand** | **Chr From** | **Probe X** | **Probe Y** | **Affy Probe Set Name** |
|---|---|---|---|---|---|---|---|
| SLC25A24 | 29957_at | 1 | - | 108677965 | 628 | 19 | 204342_at |
| SLC25A24 | 29957_at | 1 | - | 108677914 | 303 | 521 | 204342_at |
| SLC25A24 | 29957_at | 1 | - | 108677901 | 35 | 1123 | 204342_at |
| SLC25A24 | 29957_at | 1 | - | 108677869 | 275 | 637 | 204342_at |
| SLC25A24 | 29957_at | 1 | - | 108677831 | 496 | 63 | 204342_at |
| SLC25A24 | 29957_at | 1 | - | 108677774 | 905 | 679 | 204342_at |
| SLC25A24 | 29957_at | 1 | - | 108677706 | 543 | 827 | 204342_at |
| SLC25A24 | 29957_at | 1 | - | 108677696 | 242 | 325 | 204342_at |
| SLC25A24 | 29957_at | 1 | - | 108677574 | 147 | 829 | 204342_at |
| SLC25A24 | 29957_at | 1 | - | 108677559 | 381 | 923 | 204342_at |
| SLC25A24 | 29957_at | 1 | - | 108677468 | 313 | 551 | 204342_at |
| BIK | 638_at | 22 | + | 43524558 | 743 | 715 | 205780_at |
| BIK | 638_at | 22 | + | 43524597 | 400 | 939 | 205780_at |
| BIK | 638_at | 22 | + | 43525221 | 341 | 759 | 205780_at |
| BIK | 638_at | 22 | + | 43525373 | 140 | 481 | 205780_at |
| BIK | 638_at | 22 | + | 43525398 | 321 | 713 | 205780_at |
| BIK | 638_at | 22 | + | 43525425 | 575 | 215 | 205780_at |
| BIK | 638_at | 22 | + | 43525521 | 720 | 449 | 205780_at |
| BIK | 638_at | 22 | + | 43525536 | 964 | 1153 | 205780_at |
| BIK | 638_at | 22 | + | 43525592 | 975 | 469 | 205780_at |
| C9orf64 | 84267_at | 9 | - | 86554040 | 157 | 773 | 223697_x_at |
| C9orf64 | 84267_at | 9 | - | 86553955 | 1083 | 193 | 223697_x_at |
| C9orf64 | 84267_at | 9 | - | 86553930 | 326 | 909 | 223697_x_at |
| C9orf64 | 84267_at | 9 | - | 86553806 | 1002 | 1119 | 223697_x_at |
| C9orf64 | 84267_at | 9 | - | 86553792 | 37 | 899 | 223697_x_at |
| C9orf64 | 84267_at | 9 | - | 86553775 | 574 | 465 | 223697_x_at |
| C9orf64 | 84267_at | 9 | - | 86553647 | 669 | 219 | 235940_at |
| C9orf64 | 84267_at | 9 | - | 86553634 | 844 | 181 | 235940_at |
| C9orf64 | 84267_at | 9 | - | 86553627 | 533 | 1077 | 235940_at |
| C9orf64 | 84267_at | 9 | - | 86553608 | 680 | 351 | 235940_at |
| C9orf64 | 84267_at | 9 | - | 86553556 | 1004 | 857 | 235940_at |
| C9orf64 | 84267_at | 9 | - | 86553508 | 473 | 287 | 235940_at |
| C9orf64 | 84267_at | 9 | - | 86553494 | 800 | 279 | 235940_at |
| C9orf64 | 84267_at | 9 | - | 86553449 | 625 | 399 | 235940_at |
| C9orf64 | 84267_at | 9 | - | 86553424 | 620 | 15 | 235940_at |
| C9orf64 | 84267_at | 9 | - | 86553376 | 81 | 687 | 235940_at |
| PRKAR2B | 5577_at | 7 | + | 106801265 | 917 | 1109 | 203680_at |
| PRKAR2B | 5577_at | 7 | + | 106801280 | 787 | 685 | 203680_at |
| PRKAR2B | 5577_at | 7 | + | 106801383 | 881 | 11 | 203680_at |
| PRKAR2B | 5577_at | 7 | + | 106801470 | 1009 | 1055 | 203680_at |
| PRKAR2B | 5577_at | 7 | + | 106801494 | 1147 | 1067 | 203680_at |
| PRKAR2B | 5577_at | 7 | + | 106801733 | 727 | 125 | 203680_at |
| PRKAR2B | 5577_at | 7 | + | 106801749 | 1001 | 831 | 203680_at |
| LGALS 1 | 3956_at | 22 | + | 38072999 | 1117 | 755 | 201105_at |
| LGALS 1 | 3956_at | 22 | + | 38073020 | 607 | 215 | 201105_at |
| LGALS 1 | 3956_at | 22 | + | 38073031 | 672 | 761 | 201105_at |
| LGALS 1 | 3956_at | 22 | + | 38074579 | 672 | 919 | 201105_at |
| LGALS 1 | 3956_at | 22 | + | 38074611 | 994 | 157 | 201105_at |
| LGALS 1 | 3956_at | 22 | + | 38075639 | 97 | 621 | 201105_at |
| LGALS 1 | 3956_at | 22 | + | 38075707 | 749 | 183 | 201105_at |
| LHFP | 10186_at | 13 | - | 39917555 | 575 | 181 | 218656_s_at |
| LHFP | 10186_at | 13 | - | 39917535 | 335 | 465 | 218656_s_at |
| LHFP | 10186_at | 13 | - | 39917516 | 1014 | 239 | 218656_s_at |
| LHFP | 10186_at | 13 | - | 39917496 | 798 | 403 | 218656_s_at |
| LHFP | 10186_at | 13 | - | 39917326 | 75 | 1131 | 218656_s_at |
| LHFP | 10186_at | 13 | - | 39917297 | 276 | 147 | 218656_s_at |
| LHFP | 10186_at | 13 | - | 39917250 | 395 | 731 | 218656_s_at |
| LHFP | 10186_at | 13 | - | 39917222 | 43 | 585 | 218656_s_at |
| LHFP | 10186_at | 13 | - | 39917147 | 1147 | 747 | 218656_s_at |
| LHFP | 10186_at | 13 | - | 39917130 | 950 | 1011 | 218656_s_at |
| LHFP | 10186_at | 13 | - | 39917108 | 230 | 965 | 218656_s_at |
| IGHM | 3507_at | 14 | - | 106322260 | 799 | 899 | 214916_x_at |
| IGHM | 3507_at | 14 | - | 106320745 | 250 | 321 | 209374_s_at |
| IGHM | 3507_at | 14 | - | 106320708 | 396 | 617 | 209374_s_at |
| IGHM | 3507_at | 14 | - | 106320697 | 848 | 527 | 209374_s_at |
| IGHM | 3507_at | 14 | - | 106320691 | 637 | 673 | 209374_s_at |
| IGHM | 3507_at | 14 | - | 106320676 | 1079 | 487 | 209374_s_at |
| IGHM | 3507_at | 14 | - | 106320666 | 917 | 437 | 209374_s_at |
| IGHM | 3507_at | 14 | - | 106320598 | 429 | 345 | 209374_s_at |
| IGHM | 3507_at | 14 | - | 106320592 | 785 | 617 | 209374_s_at |
| IGHM | 3507_at | 14 | - | 106320527 | 810 | 967 | 209374_s_at |
| IGHM | 3507_at | 14 | - | 106320516 | 212 | 745 | 209374_s_at |
| IGHM | 3507_at | 14 | - | 106320511 | 797 | 413 | 209374_s_at |
| TAF9 | 6880_at | 5 | - | 68661140 | 1034 | 223 | 202168_at |
| TAF9 | 6880_at | 5 | - | 68661108 | 502 | 757 | 202168_at |
| TAF9 | 6880_at | 5 | - | 68661018 | 444 | 209 | 202168_at |
| TAF9 | 6880_at | 5 | - | 68660977 | 1044 | 99 | 202168_at |
| TAF9 | 6880_at | 5 | - | 68660966 | 877 | 373 | 202168_at |
| TAF9 | 6880_at | 5 | - | 68660946 | 688 | 737 | 202168_at |
| TAF9 | 6880_at | 5 | - | 68660898 | 875 | 277 | 202168_at |
| TAF9 | 6880_at | 5 | - | 68660881 | 1044 | 747 | 202168_at |
| TAF9 | 6880_at | 5 | - | 68660757 | 757 | 681 | 202168_at |
| TAF9 | 6880_at | 5 | - | 68660631 | 871 | 225 | 202168_at |
| TAF9 | 6880_at | 5 | - | 68647993 | 144 | 125 | 203893_at |
| TAF9 | 6880_at | 5 | - | 68647964 | 399 | 995 | 203893_at |
| TAF9 | 6880_at | 5 | - | 68647951 | 641 | 487 | 203893_at |
| TAF9 | 6880_at | 5 | - | 68647856 | 393 | 881 | 203893_at |
| TAF9 | 6880_at | 5 | - | 68647839 | 999 | 1027 | 203893_at |
| TAF9 | 6880_at | 5 | - | 68647828 | 1043 | 685 | 203893_at |
| TAF9 | 6880_at | 5 | - | 68647773 | 574 | 39 | 203893_at |
| PLCL2 | 23228_at | 3 | + | 17056348 | 20 | 141 | 216218_s_at |
| PLCL2 | 23228_at | 3 | + | 17056359 | 103 | 3 | 216218_s_at |
| PLCL2 | 23228_at | 3 | + | 17056379 | 1042 | 565 | 216218_s_at |
| PLCL2 | 23228_at | 3 | + | 17084370 | 1143 | 1129 | 216218_s_at |
| PLCL2 | 23228_at | 3 | + | 17084415 | 472 | 223 | 216218_s_at |
| PLCL2 | 23228_at | 3 | + | 17109453 | 340 | 549 | 216218_s_at |
| PLCL2 | 23228_at | 3 | + | 17109462 | 982 | 581 | 216218_s_at |
| PLCL2 | 23228_at | 3 | + | 17109470 | 660 | 539 | 216218_s_at |
| PLCL2 | 23228_at | 3 | + | 17109518 | 674 | 133 | 216218_s_at |
| PLCL2 | 23228_at | 3 | + | 17109529 | 173 | 515 | 216218_s_at |
| PLCL2 | 23228_at | 3 | + | 17131556 | 769 | 725 | 213309_at |
| PLCL2 | 23228_at | 3 | + | 17131644 | 262 | 607 | 213309_at |
| PLCL2 | 23228_at | 3 | + | 17131676 | 158 | 15 | 213309_at |
| PLCL2 | 23228_at | 3 | + | 17131816 | 207 | 651 | 213309_at |
| PLCL2 | 23228_at | 3 | + | 17131832 | 807 | 669 | 213309_at |
| PLCL2 | 23228_at | 3 | + | 17131844 | 365 | 877 | 213309_at |
| PLCL2 | 23228_at | 3 | + | 17131878 | 782 | 925 | 213309_at |
| PLCL2 | 23228_at | 3 | + | 17131907 | 160 | 809 | 213309_at |
| PLCL2 | 23228_at | 3 | + | 17131936 | 355 | 955 | 213309_at |
| PLCL2 | 23228_at | 3 | + | 17131951 | 1024 | 505 | 213309_at |
| PLCL2 | 23228_at | 3 | + | 17131972 | 230 | 483 | 213309_at |
| TFDP2 | 7029_at | 3 | | 141682706 | 762 | 1007 | 203588_s_at |
| TFDP2 | 7029_at | 3 | | 141678591 | 767 | 67 | 203588_s_at |
| TFDP2 | 7029_at | 3 | | 141678571 | 167 | 847 | 203588_s_at |
| TFDP2 | 7029_at | 3 | | 141678542 | 897 | 949 | 203588_s_at |
| TFDP2 | 7029_at | 3 | | 141671834 | 924 | 23 | 203588_s_at |
| TFDP2 | 7029_at | 3 | | 141671806 | 125 | 1021 | 203588_s_at |
| TFDP2 | 7029_at | 3 | | 141671769 | 719 | 287 | 203588_s_at |
| TFDP2 | 7029_at | 3 | | 141671484 | 201 | 91 | 203588_s_at |
| TFDP2 | 7029_at | 3 | | 141671452 | 1047 | 303 | 203588_s_at |
| TFDP2 | 7029_at | 3 | | 141671350 | 1088 | 5 | 203588_s_at |
| TFDP2 | 7029_at | 3 | | 141670849 | 300 | 805 | 203589_s_at |
| TFDP2 | 7029_at | 3 | | 141670821 | 1147 | 813 | 203589_s_at |
| TFDP2 | 7029_at | 3 | | 141670782 | 1003 | 59 | 203589_s_at |
| TFDP2 | 7029_at | 3 | | 141670774 | 1099 | 365 | 203589_s_at |
| TFDP2 | 7029_at | 3 | | 141670739 | 524 | 79 | 203589_s_at |
| TFDP2 | 7029_at | 3 | | 141670648 | 960 | 549 | 203589_s_at |
| TFDP2 | 7029_at | 3 | | 141670597 | 856 | 519 | 203589_s_at |
| TFDP2 | 7029_at | 3 | | 141670572 | 114 | 367 | 203589_s_at |
| TFDP2 | 7029_at | 3 | | 141670451 | 489 | 623 | 203589_s_at |
| TFDP2 | 7029_at | 3 | | 141670430 | 834 | 1141 | 203589_s_at |
| TFDP2 | 7029_at | 3 | | 141670415 | 537 | 725 | 203589_s_at |
| TFDP2 | 7029_at | 3 | | 141669240 | 136 | 591 | 244043_at |
| TFDP2 | 7029_at | 3 | | 141669231 | 458 | 303 | 244043_at |
| TFDP2 | 7029_at | 3 | | 141669196 | 80 | 471 | 244043_at |
| TFDP2 | 7029_at | 3 | | 141669177 | 1092 | 917 | 244043_at |
| TFDP2 | 7029_at | 3 | | 141669152 | 231 | 359 | 244043_at |
| TFDP2 | 7029_at | 3 | | 141669139 | 412 | 777 | 244043_at |
| TFDP2 | 7029_at | 3 | | 141669100 | 817 | 1019 | 244043_at |
| TFDP2 | 7029_at | 3 | | 141669082 | 217 | 757 | 244043_at |
| TFDP2 | 7029_at | 3 | | 141669056 | 659 | 587 | 244043_at |
| TFDP2 | 7029_at | 3 | | 141669041 | 579 | 931 | 244043_at |
| TFDP2 | 7029_at | 3 | | 141668997 | 1134 | 45 | 244043_at |
| TFDP2 | 7029_at | 3 | | 141663898 | 95 | 277 | 226157_at |
| TFDP2 | 7029 art | 3 | | 141663820 | 688 | 47 | 226157_at |
| TFDP2 | 7029_at | 3 | | 141663770 | 564 | 275 | 226157_at |
| TFDP2 | 7029_at | 3 | | 141663753 | 917 | 945 | 226157_at |
| TFDP2 | 7029_at | 3 | | 141663614 | 108 | 1059 | 226157_at |
| TFDP2 | 7029_at | 3 | | 141663568 | 68 | 599 | 226157_at |
| TFDP2 | 7029_at | 3 | | 141663549 | 1060 | 973 | 226157_at |
| TFDP2 | 7029_at | 3 | | 141663521 | 645 | 483 | 226157_at |
| TFDP2 | 7029_at | 3 | | 141663502 | 143 | 549 | 226157_at |
| TFDP2 | 7029_at | 3 | | 141663472 | 33 | 757 | 226157_at |
| TFDP2 | 7029_at | 3 | | 141663388 | 603 | 93 | 226157_at |
| LIG4 | 3981_at | 13 | | 108861141 | 1046 | 713 | 206235_at |
| LIG4 | 3981_at | 13 | | 108861124 | 438 | 965 | 206235_at |
| LIG4 | 3981_at | 13 | | 108861095 | 407 | 59 | 206235_at |
| LIG4 | 3981_at | 13 | | 108861071 | 659 | 245 | 206235_at |
| LIG4 | 3981_at | 13 | | 108861063 | 930 | 627 | 206235_at |
| LIG4 | 3981_at | 13 | | 108861036 | 151 | 715 | 206235_at |
| LIG4 | 3981_at | 13 | | 108861019 | 655 | 803 | 206235_at |
| LIG4 | 3981_at | 13 | | 108860997 | 984 | 813 | 206235_at |
| LIG4 | 3981_at | 13 | | 108860837 | 467 | 591 | 206235_at |
| LIG4 | 3981_at | 13 | | 108860822 | 31 | 875 | 206235_at |
| LIG4 | 3981_at | 13 | | 108860696 | 1079 | 583 | 206235_at |
| LIG4 | 3981_at | 13 | | 108860199 | 1136 | 1043 | 227766_at |
| LIG4 | 3981_at | 13 | | 108860139 | 342 | 1117 | 227766_at |
| LIG4 | 3981_at | 13 | | 108860127 | 453 | 519 | 227766_at |
| LIG4 | 3981_at | 13 | | 108860073 | 572 | 551 | 227766_at |
| LIG4 | 3981_at | 13 | | 108860059 | 344 | 71 | 227766_at |
| LIG4 | 3981_at | 13 | | 108859941 | 41 | 289 | 227766_at |
| LIG4 | 3981_at | 13 | | 108859931 | 71 | 1053 | 227766_at |
| LIG4 | 3981_at | 13 | | 108859907 | 329 | 593 | 227766_at |
| LIG4 | 3981_at | 13 | | 108859892 | 1017 | 935 | 227766_at |
| LIG4 | 3981_at | 13 | | 108859884 | 993 | 491 | 227766_at |
| LIG4 | 3981_at | 13 | | 108859846 | 804 | 1105 | 227766_at |

### Example 2 - Validation using an independent clinical test sample set

In the validation stage, the newly identified gene expression signature was used in the analysis focusing the clinical patient samples, using the unsupervised hierarchical clustering analysis. The data used in this analysis was obtained from the publically available data repositories, i.e. Gene Expression Omnibus (GEO) [Barrett T, et al., NCBI GEO: archive for functional genomics data sets--update. Nucleic Acids Res. 27 November 2012;41(D1):D991-D995], originally analyzed and published by Hummel et al. [supra]. The identified gene expression signature enabled highly specific and sensitive clustering of the analyzed lymphoma samples. As the result, two distinct clusters were observed: one consisting almost only of DLBCL patients, and the other one which was almost entirely comprised of well-defined BL samples, including vast majority of the cases clinically diagnosed as aBL, i.e. those that are most difficult in unambiguous classification using traditionally used histopathological methods. Importantly, as compared with the clustering based on the minimal set of four genes common between the Hummel et al. 58 gene signature and the 11 gene signature identified in our training data analysis (Figure 5A), addition of the 7 newly identified genes (4 + 7) enabled us to cluster the clinical cases of BL together with aBL with both increased specificity of 95% and sensitivity of 74% (Figure 5B) with assumption of the histopathological assessment being the golden standard in the diagnostics of B-cell lymphomas.

This indicates that the newly identified gene hits indeed have the classification potential not only in the cell line material but also in the clinical sample setup and may include genes which are important for the biology of either of the B-cell lymphoma types.

### Example 3 - Relative expression in lymphoma cell lines by RT-qPCR

The selected genes derived from the identified gene expression signature were tested for their differential expression between DLBCL and Burkitt's lymphomas using the independent reverse-transcribed mRNA (cDNA) samples extracted from an array of cell lines: Bjab, Daudi, DHL4, DHL6, Ly10, Ly19, Ly4, Ly7, Namalwa, Raji, Ramos, U2932.

Primer sequences (Table 3) were designed using the Primer3 algorithm [Rozen S and Skaletsky H., Primer3 on the WWW for General Users and for Biologist Programmers. W: Misener S and Krawetz SA, eds. Bioinformatics Methods and Protocols (Internet). Humana Press; 1999 (cited 8 July 2014]. p. 365-86. Downloaded from: http://link.springer.com/protocol/10.1385/1-59259-192-2%3A365]. In order to maintain the maximum specificity of the primer pairs towards its transcript targets each pair of primers included one primer spanning an exon-exon junction. The qPCR reactions were done using the LightCycler^{®}480 Instrument II from Roche Applied Science.

**Table 3 Sequences of primers used for relative expression quantification (RT-qPCR) of the selected genes in an independent set of lymphoma cell lines.**

| **Gene Name** | **Primer Name** | **Sequence 5' → 3'** |
|---|---|---|
| C9orf64 | orf64_5F | CAGAGATGTGACTCTGTTTGAGG (SEQ NO ID: 1) |
| C9orf64 | orf64_5R | AGCAAACATGGTGATACTGGAG (SEQ NO ID: 2) |
| LHFP | LHFP_2F | GGCTTGTTGATTGGTGCTGG (SEQ NO ID: 3) |
| LHFP | LHFP_2R | CACACTTCCCCAGGTCAAACT (SEQ NO ID: 4) |
| PRKAR2B | PRK2B_4F | CTGCTACCTCTCCTGGTGCT (SEQ NO ID: 5) |
| PRKAR2B | PRK2B_4R | TTTTTGGCATTGTTTTTCACA (SEQ NO ID: 6) |
| ACTB | ACTB_F | TTCCTTCCTGGGCATGGAGT (SEQ NO ID: 7) |
| ACTB | ACTB_R | ATCCACATCTGCTGGAAGGT (SEQ NO ID: 8) |

The results of the RT-qPCR validation of our gene expression signature hits have shown that the relative expression of the selected genes is fully concordant with results of the bioinformatics stage of the present invention as shown on Figure 6.

The results of the above studies have demonstrated that the set of 11 carefully selected genes has robust classificatory properties for stratification of the B-cell malignancies into groups requiring different therapeutic strategies in the clinical setup. By facilitating a proper diagnostics application of the described approach can translate into improved survival of the patients diagnosed with either Burkitt's lymphomas or the Diffuse Large B-cell lymphomas.

### Example 4 - Use of gene expression signature of the invention to determine probability of Burkitt's lymphoma diagnosis

The applicability of the gene expression signature of the invention to determine the probability of Burkitt's lymphoma or DLBCL diagnosis is based on use of information conveyed by the transcription levels of the signature-selected genes in computation of the probability coefficients, at which newly diagnosed patients can be assigned to either Burkitt's lymphoma (including the atypical cases of Burkitt's lymphoma) or the DLBCL group.

To achieve this a training group of patients containing both well-defined cases of Burkitt's lymphoma, as well as confirmed DLBCL cases should be assayed against all of the 11 genes of the gene expression signature of the invention. Additionally, the training group of patients should also be assayed for at least one reference gene (for example, human ACTB - actin, beta gene), which reference gene(s) are used for normalization of the raw gene expression signal of the 11 signature-selected genes of the invention. The measurements of the gene expression levels for the targeted genes can be conveniently done using a custom-made platform involving lyophilized qPCR (Quantitative Polymerase Chain Reaction) primers and probes in the format of LDA (Low Density Array) 384-well plates.

Such data is then used to build a statistical model (logistic regression - with DLBCL category being the base level of the dependent variable) precisely describing the relationships between the normalized gene expression for the selected 11 genes of the gene expression signature of the invention and the clinical outcomes in the training group patients.

The constructed logistic regression model is then used in algorithms involving principles of machine learning to predict probability of the clinical outcomes in newly recruited patients suffering from suspected lymphoma.

In order to compute the probability of Burkitt's lymphoma diagnosis the fresh-frozen-preserved biopsy samples of the newly recruited patients are assayed against the same selection of 11 signature genes of the invention and the reference genes, as those assayed in the training group of patients, using exactly the same methodology. The obtained gene expression profiles may be then used to apply prediction algorithms to compute the probability of Burkitt's lymphoma diagnosis.

To illustrate the effect of the prediction algorithm, the Inventors used microarray data of normalized (RMA - Robust Multiarray Average) gene expression profiles of randomly selected 15 patients from the data set published by Hummel M et al., [supra]. Both logistic regression modeling, as well as computation of prediction coefficients was carried out in R-project statistical environment using glm() and predict.glm() functions available in the base {stats} distribution of R-project. The calculated probability of Burkitt's lymphoma are summerized in Table 4 below.

**Table 4 Simulated computation of probability of Burkitt's lymphoma diagnosis for the 15 randomly selected patients morphologically described as Burkitt's lymphoma or DLBCL in the clinical setting.**

| **Patient id** | **Clinical morphology** | **Probability of Burkitt's lymphoma** |
|---|---|---|
| MPI-017 | BL | 0.778 |
| MPI-089 | BL | 0.87 |
| MPI-079 | atypical BL | 0.793 |
| MPI-173 | atypical BL | 0.005 |
| MPI-085 | atypical BL | 0.957 |
| MPI-054 | atypical BL | 0.013 |
| MPI-030 | DLBCL | 0.011 |
| MPI-031 | DLBCL | 0.015 |
| MPI-200 | DLBCL | 0.004 |
| MPI-129 | DLBCL | 0.006 |
| MPI-149 | DLBCL | 0.045 |
| MPI-161 | DLBCL | 0.032 |
| MPI-162 | DLBCL | 0.01 |
| MPI-154 | DLBCL | 0.001 |
| MPI-188 | DLBCL | 0.034 |

The estimations of the probability of Burkitt's lymphoma obtained using the aforementioned methodology (both laboratory and statistical) in concert with traditionally used diagnostic tools may become a valuable tool facilitating improved diagnostics, especially towards the difficult cases of atypical Burkitt's lymphoma cases which otherwise are difficult to detect in a clinical setting.

## Claims

1. Use of gene expression signature consisting of SLC25A24, BIK, C9orf64, PRKAR2B, LGALS1, LHFP, IGHM, TAF9, PLCL2, TFDP2 and LIG4 for differentiation between Burkitt's lymphoma (BL) and diffuse large B-cell lymphoma (DLBCL).

2. The qPCR kit for differentiation between BL and DLBCL, **characterized in that** it consists of primers for the combination of the genes consisting of SLC25A24, BIK, C9orf64, PRKAR2B, LGALS1, LHFP, IGHM, TAF9, PLCL2, TFDP2 and LIG4 and primers for at least one reference gene.

3. The kit of claim 2, wherein the kit comprises primers selected from a group consisting of SEQ NO ID: 1 to 8.

4. A microarray for use in differentiation between BL and DLBCL consisting of probes for identification of SLC25A24, BIK, C9orf64, PRKAR2B, LGALS1, LHFP, IGHM, TAF9, PLCL2, TFDP2 and LIG4 genes and probes for at least one reference gene.

5. An *in vitro* method of differentiation between Burkitt's lymphoma (BL) and diffuse large B-cell lymphoma (DLBCL), **characterized in that** it comprises the steps of:
a) obtaining the gene expression profile consisting of the following genes: SLC25A24, BIK, C9orf64, PRKAR2B, LGALS1, LHFP, IGHM, TAF9, PLCL2, TFDP2, and LIG4, for samples from a training group of patients diagnosed as suffering from BL or DLBCL, wherein the gene expression profile is normalized based on the expression level of at least one reference gene;
b) obtaining the gene expression profile consisting of the following genes: SLC25A24, BIK, C9orf64, PRKAR2B, LGALS1, LHFP, IGHM, TAF9, PLCL2, TFDP2, and LIG4, for a sample from a patient suffering from non-Hodgkin lymphoma (NHL) to be differentiated as either BL and DLBCL, wherein the gene expression profile is normalized based on the expression level of at least one reference gene and wherein the processes of obtaining the gene expression profile and its normalization are carried out in the same way as in step (a);
c) calculating the probability of BL and DLBCL diagnosis for a sample from step (b) based on an algorithm designed in reference to the data obtained in step (a).

## Patentansprüche

1. Verwendung von Signatur der Genexpression bestehend aus SLC25A24, BIK, C9orf64, PRKAR2B, LGALS1, LHFP, IGHM, TAF9, PLCL2, TFDP2 und LIG4 zur Unterscheidung zwischen Burkitt-Lymphom (BL) und diffusem großzelligen B-Zell-Lymphom (DLBCL).

2. Das qPCR-Kit zur Unterscheidung zwischen BL und DLBCL, **dadurch gekennzeichnet, dass** es aus Primern für die Kombination der Gene bestehend aus SLC25A24, BIK, C9orf64, PRKAR2B, LGALS1, LHFP, IGHM, TAF9, PLCL2, TFDP2 und LIG4 und Primern für mindestens ein Referenzgen besteht.

3. Kit nach Anspruch 2, wobei der Kit die Primer ausgewählt aus einer Gruppe bestehend aus SEQ NO ID: 1 bis 8 umfasst.

4. Das Microarray zur Verwendung in Unterscheidung zwischen BL und DLBCL bestehend aus Sonden zur Identifikation von SLC25A24, BIK, C9orf64, PRKAR2B, LGALS1, LHFP, IGHM, TAF9, PLCL2, TFDP2 und LIG4 Genen und Sonden für mindestens ein Referenzgen.

5. Ein *in vitro* Verfahren zur Unterscheidung zwischen Burkitt-Lymphom (BL) und diffusem großzelligen B-Zell-Lymphom (DLBCL), **dadurch gekennzeichnet, dass** es die folgenden Schritte umfasst:
a) Erhalten des Genexpressionsprofils, bestehend aus den folgenden Genen: SLC25A24, BIK, C9orf64, PRKAR2B, LGALS1, LHFP, IGHM, TAF9, PLCL2, TFDP2, und LIG4 für Proben aus einer Trainingsgruppe von Patienten, die als BL oder DLBCL diagnostiziert wurden, wobei das Genexpressionsprofil auf der Grundlage des Expressionsniveaus von mindestens einem Referenzgen normalisiert wird;
b) Erhalten des Genexpressionsprofils, bestehend aus den folgenden Genen: SLC25A24, BIK, C9orf64, PRKAR2B, LGALS1, LHFP, IGHM, TAF9, PLCL2, TFDP2, und LIG4 für eine Probe von einem Patienten, der an Non-Hodgkin-Lymphom (NHL) leidet, um entweder als BL und DLBCL differenziert zu sein, wobei das Genexpressionsprofil auf der Grundlage des Expressionsniveaus von mindestens einem Referenzgen normalisiert wird und wobei die Prozesse des Erhaltens des Genexpressionsprofils und seine Normalisierung auf die gleiche Weise wie in Schritt (a) durchgeführt werden;
c) Berechnen der Wahrscheinlichkeit der BL- und DLBCL-Diagnose für eine Probe aus dem Schritt (b) basierend auf einem Algorithmus, der in Bezug auf die im Schritt (a) erhaltenen Daten entworfen wurde.

## Revendications

1. Utilisation de la signature d'expression de gène consistant en SLC25A24, BIK, C9orf64, PRKAR2B, LGALS1, LHFP, IGHM, TAF9, PLCL2, TFDP2 et LIG4 pour la différenciation entre le lymphome de Burkitt (BL) et le lymphome diffus à grandes cellules B (DLBCL).

2. Kit qPCR pour la différenciation entre BL et DLBCL, **caractérisé en ce qu'**il consiste en des amorces pour la combinaison des gènes consistant en SLC25A24, BIK, C9orf64, PRKAR2B, LGALS1, LHFP, IGHM, TAF9, PLCL2, TFDP2 et LIG4 et des amorces pour au moins un gène de référence.

3. Kit selon la revendication 2, dans lequel le kit comprend des amorces sélectionnées dans un groupe constitué de SEQ NO ID : 1 à 8.

4. Microréseau à utiliser dans la différenciation entre BL et DLBCL, consistant en des sondes pour l'identification des gènes SLC25A24, BIK, C9orf64, PRKAR2B, LGALS1, LHFP, IGHM, TAF9, PLCL2, TFDP2 et LIG4 et des sondes pour au moins un gène de référence.

5. Méthode *in vitro* de différenciation entre le lymphome de Burkitt (BL) et le lymphome diffus à grandes cellules B (DLBCL), **caractérisée en ce qu'**elle comprend les étapes consistant à :
a) obtenir le profil d'expression de gène consistant en les gènes suivants : SLC25A24, BIK, C9orf64, PRKAR2B, LGALS1, LHFP, IGHM, TAF9, PLCL2, TFDP2 et LIG4, pour des échantillons d'un groupe de formation de patients diagnostiqués comme souffrant de BL ou DLBCL, dans laquelle le profil d'expression de gène est normalisé en fonction du niveau d'expression d'au moins un gène de référence;
b) obtenir le profil d'expression de gène consistant en les gènes suivants : SLC25A24, BIK, C9orf64, PRKAR2B, LGALS1, LHFP, IGHM, TAF9, PLCL2, TFDP2 et LIG4, pour un échantillon d'un patient souffrant de lymphome non-Hodgkinien (NHL) à différencier comme étant soit BL, soit DLBCL, dans laquelle le profil d'expression de gène est normalisé en fonction du niveau d'expression d'au moins un gène de référence et dans laquelle les processus d'obtention du profil d'expression de gène et sa normalisation sont effectués de la même manière que dans l'étape (a)
c) calculer la probabilité de diagnostic BL et DLBCL pour un échantillon de l'étape (b) sur la base d'un algorithme conçu en référence aux données obtenues dans l'étape (a).


### SEQUENCE LISTING

<110> Warszawski Uniwersytet Medyczny
<120> Stratification of B-cell lymphoma cases using a gene expression signature
<130> P30919EP00
<160> 8
<170> PatentIn version 3.5
<210> 1
   <211> 23
   <212> DNA
   <213> Artificial Sequence
<220>
   <223> orf64_5F primer
<400> 1
   cagagatgtg actctgtttg agg 23
<210> 2
   <211> 22
   <212> DNA
   <213> Artificial Sequence
<220>
   <223> orf64_5R primer
<400> 2
   agcaaacatg gtgatactgg ag 22
<210> 3
   <211> 20
   <212> DNA
   <213> Artificial Sequence
<220>
   <223> LHFP_2F primer
<400> 3
   ggcttgttga ttggtgctgg 20
<210> 4
   <211> 21
   <212> DNA
   <213> Artificial Sequence
<220>
   <223> LHFP_2R primer
<400> 4
   cacacttccc caggtcaaac t 21
<210> 5
   <211> 20
   <212> DNA
   <213> Artificial Sequence
<220>
   <223> PRK2B_4F primer
<400> 5
   ctgctacctc tcctggtgct 20
<210> 6
   <211> 21
   <212> DNA
   <213> Artificial Sequence
<220>
   <223> PRK2B_4R primer
<400> 6
   tttttggcat tgtttttcac a 21
<210> 7
   <211> 20
   <212> DNA
   <213> Artificial Sequence
<220>
   <223> ACTB_F primer
<400> 7
   ttccttcctg ggcatggagt 20
<210> 8
   <211> 20
   <212> DNA
   <213> Artificial Sequence
<220>
   <223> ACTB_R primer
<400> 8
   atccacatct gctggaaggt 20